# EUROPEAN PATENT APPLICATION

(11) **EP 1 491 215 A1**
(43) Date of publication of application: **29.12.2004**
(21) Application number: 03745404.8
(22) Date of filing: 05.03.2003
(51) Int. Cl.: A61K 48/00, A61K 45/00, A61K 9/51, A61K 9/08, A61K 38/45, A61P 1/16, A61P 35/00

(54) **REMEDIES WITH THE USE OF HOLLOW PROTEIN NANOPARTICLES PRESENTING GROWTH FACTOR OR THE LIKE**

(30) Priority: 29.03.2002 JP 2002097395
(71) Applicant: Japan Science and Technology Agency, Kawaguchi-shi, Saitama 332-0012 (JP)
(72) Inventor: KURODA, Shunichi, Suita-shi, Osaka 565-0872 (JP); TANIZAWA, Katsuyuki, Toyono-gun, Osaka 563-0214 (JP); KONDO, Akihiko, Kobe-shi, Hyogo 657-0015 (JP); UEDA, Masakazu, Shinjuku-ku, Tokyo 162-0837 (JP); SENO, Masaharu, Okayama-shi, Okayama 703-8273 (JP); IWABUKI, Hidehiko, Onsen-gun, Ehime 791-0303 (JP)
(74) Representative: Bentz, Jean-Paul
(86) International application number: PCT/JP2003/002601
(87) International publication number: WO 2003/082344

(57) **Abstract**

The invention provides a disease-treating drug, specifically acting particular cells or tissues, which is based on protein hollow nanoparticles. The drug's therapeutic effects are confirmed through animal experiments. The invention also provides a treatment method using the drug. The disease-treating drug comprises a substance to be transferred into a cell for treatment of a disease (for example, cancer-treating, thymidine kinase gene of herpes simplex virus type 1) encapsulated in hollow nanoparticles containing particle-forming protein (for example, hepatitis B virus surface antigen protein modified to lose infectivity to inherent hepatocyte and also display a growth factor) displaying, for example, a growth factor.

## Description

### TECHNICAL FIELD

The present invention relates to therapeutic drugs based on protein hollow nanoparticles displaying growth factor, etc., in particular, drugs which contain particles encapsulating a disease-treating substance to be transferred into a cell and are capable of specifically transferring the substance to be transferred into a cell to particular cells or tissues.

### BACKGROUND ART

In recent years, in the field of medicine, development is actively pursued of highly effective drugs acting directly on an affected part with less side effect. Especially, a method known as the drug delivery system (DDS) has attracted much attention since it allows for the transport of drugs and other effective components to specific target cells or tissues and enables the effective components to act at the target site.

Also, in the field of recent molecular cell biology, gene transfer into particular cells has been recognized as an essential technology and being actively studied. Further, with the recent discoveries in the genetic background of various diseases by the progress of the Human Genome Project, the realization of such highly specific methods for gene transfer into cells and tissues would also enable application in the field of gene therapy.

Conventional methods of transferring a gene into cells include a method of incorporating a macromolecular form of gene by endocytosis (calcium phosphate method, lipofectamine method, etc.) and a method of transferring a gene into cells by perforating the cell membrane with electrical pulse stimulation (electroporation or gene gun method). All of these techniques are now commonly used in molecular biology experiments.

Although being simple and convenient, these methods may not readily administered to cells and tissues in vivo, because they inevitably surgically expose the site where genes are transferred, incurring direct, physical damage to the cell. In addition, it is difficult to achieve a transfer efficiency close to 100%.

An alternative is the liposome method, which is a highly safe substance transfer method. The method does not damage cells and can be administered to cells and tissues in vivo. However, it is difficult to give high cell and tissue specificity to liposome, which is a simple lipid. Another problem is that the gene transfer efficiency in vivo is far lower than the required value.

Recently, a new gene transfer technique has been developed which uses an infectivity virus created by integrating a gene of interest into the virus DNA. This method can be administered to individuals with approximately 100% transfer efficiency, does not require the gene transfer site to be exposed, and thus has attracted much attention as an epoch-making method. However, there is a serious problem that the gene is transferred into cells other than the target since the virus nonspecifically infects a wide range of cells. In addition, the virus genome itself may possibly be integrated to the chromosome to induce unexpected side effect in the future. Therefore, the technique has not yet been used to treat early stages of a disease, and at present has been administered only to patients of terminus symptoms.

In view of the above situation, the same inventors as this invention suggested in International Application (published on September 7, 2001 under International Publication No. WO01/64930; hereinafter simply "International Application WO01/64930") a method of safe, specific transport and transfer of a substance (gene, protein, compound, etc.) into target cells and tissues. The method uses hollow nanoparticles containing a particle-forming protein into which are transferred biorecognition molecules. A next issue is to develop therapeutic drugs based on that method which specifically act on particular cells or tissues.

In view of this issue, the present invention has an objective to provide a disease-treating drug based on protein hollow nanoparticles which specifically act on particular cells or tissues and the therapeutic effects of which is actually confirmed through animal experiments and to provide a treatment method based on the drug.

### DISCLOSURE OF INVENTION

The inventors of the present invention have diligently worked on the issue and as a result found that by administering hepatitis B virus surface antigen particles encapsulating a cancer-treating gene and having displayed a growth factor to experimental animals transplanted with human squamous cell carcinoma through intravenous injection, cancer-treating gene is transferred specifically into the human squamous cell carcinoma cells, treating the transplanted cancer, which has led to the completion of the invention.

In other words, the drug of the present invention is a substance to be transferred into a cell for treatment of a disease encapsulated in hollow nanoparticles containing a particle-forming protein, the nanoparticles displaying molecules which bind with particular molecules on a cell surface such as a growth factor.

An example of the particle-forming protein is hepatitis B virus surface antigen protein modified to lose inherent infectivity to hepatocyte and also display, for example, growth factor. The protein, when expressed in eucaryotic cells, is expressed and accumulated as a membrane protein on an endoplasmic reticulum membrane and released as particles into the endoplasmic lumen. The hollow nanoparticles thus obtained display, for example, growth factor on their surface. Thus, the drug is capable of specifically transporting the substance in particles to particular cells. Here, the particular cells refer to, for example, those cells expressing, for example, a receptor for a growth factor on their surface, into which the substance in the hollow nanoparticles are transferred through the binding of the nanoparticles with the growth factor.

Therefore, the encapsulating of the substance (drug) for treatment of a disease into the hollow nanoparticles provides an effective therapeutic drug specifically and effectively acting on particular cells or tissues.

The molecule which binds with a particular molecule on a cell surface refers to a growth factor such as epidermal growth factor (EGF), as well as interleukin, interferon, colony stimulating factors, tumor necrosis factors, transforming growth factor β, platelet-derived growth factor, erythropoietin, Fas antigens, activin, bone morphogenetic proteins, and nerve growth factors.

The substance encapsulated in hollow nanoparticles for transfer into a cell is, for example, a cancer-treating gene. To use a drug encapsulating thymidine kinase (HSV1 tk) gene of herpes simplex virus type 1 as a cancer-treating gene, ganciclovir is administered separately as will be detailed later in Examples.

The drug of the present invention enables effective treatment of diseases of particular cells or tissues through a simple method of intravenous injection. The drug vastly differs from conventional treatment; it does not require administration of a large amount of drug or surgery as in gene therapy. It has extremely low chances of side effects and are straightly applicable in clinical practice.

The treatment method of the present invention is a treatment of diseases by the administration of the drug of the present invention.

Additional objects, advantages and novel features of the invention will be set forth in part in the description which follows, and in part will become apparent to those skilled in the art upon examination of the following or may be learned by practice of the invention.

### BRIEF DESCRIPTION OF DRAWINGS

Figure 1 is a schematic drawing showing each protein region of an HBsAg gene in the Examples of the present invention. 1 to 8 indicates the function at each site of the surface antigen.
Figure 2 is an explanatory schematic drawing showing the expression and purification procedures for HBsAg particles using recombinant yeast in an Example of the present invention as an example. Each represents: (a) Preparation of recombinant yeast; (b) Culture on a High-Pi medium; (c) Culture on an 8S5N-P400 medium; (d) Homogenization; (e) Density gradient centrifugation; and (f) HBsAg particles.
Figure 3 is a graph showing therapeutic effects of the drug of the present invention on experimental animals.
Figure 4 is a drawing an example of a substance to be transferred into a cell of the present invention.
Figure 5 is a drawing an example of a substance to be transferred into a cell of the present invention.
Figure 6 is a drawing an example of a substance to be transferred into a cell of the present invention.
Figure 7 is a drawing an example of a substance to be transferred into a cell of the present invention.
Figure 8 is a table showing therapeutic effects of the drug of the present invention on experimental animals.

### BEST MODE FOR CARRYING OUT INVENTION

The hollow nanoparticles constituting a drug of the present invention enables the specific transport of a substance to target cells or tissues expressing, for example, a receptor for a growth factor by transferring a biorecognition molecule (such as the growth factor, which is capable of binding with specific molecules on a cell surface) into a particle-forming protein. As a particle-forming protein, subviral particles obtained from a variety of viruses may be administered. Specifically, hepatitis B virus (Hepatitis B Virus, or HBV) surface antigen protein is exemplified.

The protein particles containing a particle-forming protein include those obtained by expressing the protein in an eucaryotic cell. That is, when a particle-forming protein is expressed in an eucaryotic cell, the protein is expressed and accumulated as a membrane protein on the endoplasmic reticulum membrane and is released as particles into the endoplasmic lumen. As the eucaryotic cell, yeast, insect cells, mammal cells, and like animal cells are applicable.

The present inventors have reported, as described in the following Examples, that when the L protein of the above-described HBV-surface antigen is expressed in the recombinant yeast, elliptical hollow particles of about 20 nm in diameter and about 150 nm in length, with large amounts of the protein embedded within the lipid bilayer membranes derived from yeast, were formed from the expressed HBV-surface antigen L protein (J. Biol. Chem., Vol. 267, No. 3, 1953-1961, 1992). Since these particles contain no HBV genome, they do not act as viruses and are highly safe to the human body. In addition, if the L protein of the above-described HBV-surface antigen is modified so as to lose inherent infectivity to hepatocytes, further modified so as to, for example, display a growth factor, and expressed, the particles are effective as transporters for the specific transportation of substances to cells expressing a receptor for the growth factor, because the particles display the growth factor on their surface.

The method of forming protein particles using recombinant yeast in this manner is preferred, because the particles are produced as soluble protein in cell lysates at high efficiency.

In contrast, it can be said that insect cells are eucaryotic cell closer to higher animals than yeast and are preferable for mass production of various proteins, because they can reproduce high-order structures, such as sugars, which yeast cannot reproduce. Conventional systems of insect cells utilized baculoviruses, and protein expression was accompanies by virus expression, which led to cell death and lysis when expressing protein. Consequently, there was a problem that the continuous expression of the protein occurred, and that the protein was degraded by protease released from the dead cells. Additionally, when protein is expressed and secreted, it was difficult to purify the protein secreted in a culture medium, because of contamination with a large amount of fetal bovine serum in the medium. Recently, however, an insect cell system without the use of baculovirus, which may be cultured under serum-free conditions, was developed and marketed by Invitrogen. Accordingly, by using such insect cells, it is possible to obtain protein particles that are purified easily, and in which high-order structure is reproduced.

In the protein hollow nanoparticles of the present invention, with respect to hollow nanoparticles displaying a growth factor or the like on the surface of the particles obtained by the various method described above, by transferring various substance (DNAs, RNAs, proteins, peptides, drugs, etc.) into the particles, the transportation of substances to particular cells or tissues expressing the receptor or the like for the growth factor at a considerably high specificity becomes possible.

Of course, the particle-forming protein is not limited to the modified hepatitis B virus surface antigen protein and may be any kind of proteins as long as they are able to form particles: naturally occurring proteins derived from animal cells, plant cells, viruses, fungi, or the like, as well as various synthetic proteins are applicable. Further, for example, if there is a probability that an antigen protein of viral origin induces the generation of antibodies in the living body, it may be used as a particle-forming protein after modification to reduce its antigenicity. For example, the hepatitis B virus surface antigen protein of which the antigenicity is reduced disclosed in International Application WO01/64930 is applicable. The other modified proteins disclosed in the international application (hepatitis B virus surface antigen proteins modified with gene manipulation technology) are also applicable.

Besides later detailed epidermal growth factors (EGFs), for example, interleukin, interferon, colony stimulating factors, tumor necrosis factors, transforming growth factor β, platelet-derived growth factors, erythropoietin, Fas antigens, activin, bone morphogenetic proein, and nerve growth factors are preferably used as molecules including growth factors which bind with particular molecules on a cell surface and which are displayed on a particle surface. These may be chosen properly according to the target cells or tissues.

According to the present invention, a substance desired to be transferred into target cells or tissues (a substance to be transferred into a cell) is incorporated in the protein hollow nanoparticles as described above, to form a transporter of a substance that shows cell specificity. The substance to be transferred into cells, which is incorporated in the transporter, includes: genes, such as DNAs and RNAs; natural or synthetic proteins; oligonucleotides, peptides, drugs, natural or synthetic compounds; and any other like substances.

Specifically, human RNase 1 (Jinno H., Ueda M., Ozawa S., Ikeda T., Enomoto K., Psarras K., Kitajima M., Yamada H., Seno M., Life Sci. 1996, 58 (21), 1901-8) and RNase 3 (also known as ECP: Eosinophil Cationic Protein; Mallorqui-Fernandez G., Pous J., Peracaula R., Aymami J., Maeda T., Tada H., Yamada H., Seno M., de Llorens R., Gomis-Ruth F X, Coll M., J. Mol. Biol., Jul. 28, 2000, 300 (5), 1297-307), which has been reported by the present inventors are applicable.

These proteins act inside and outside the cells and show cytotoxic activity. However, by using the transporters (drugs) of the present invention incorporating RNase, they can be detoxicated outside the cells and act only in the cells and are expected to be a new therapeutic method for cancer treatment which shows less side effect.

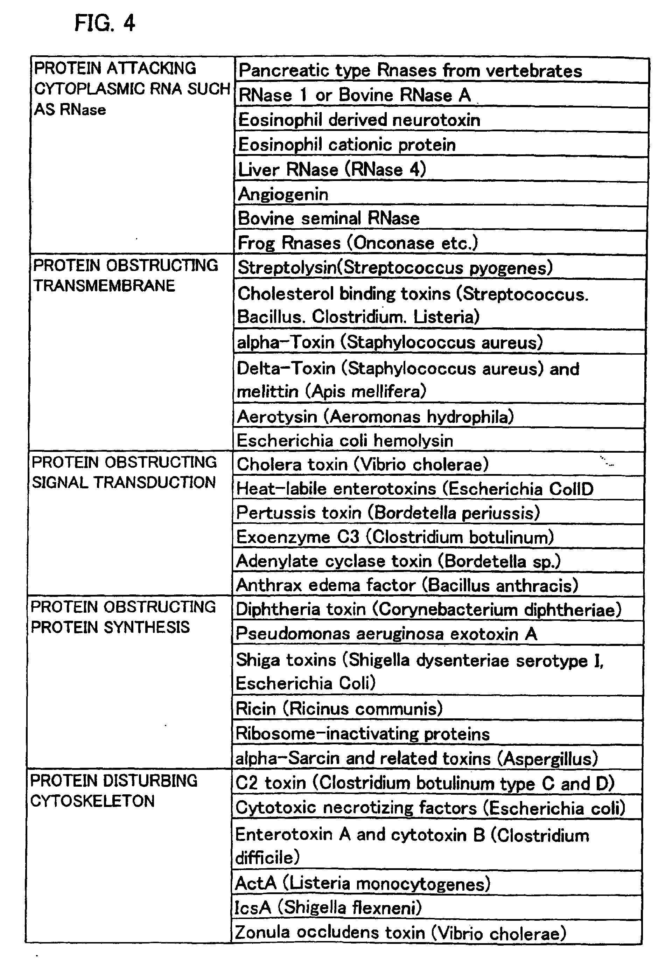

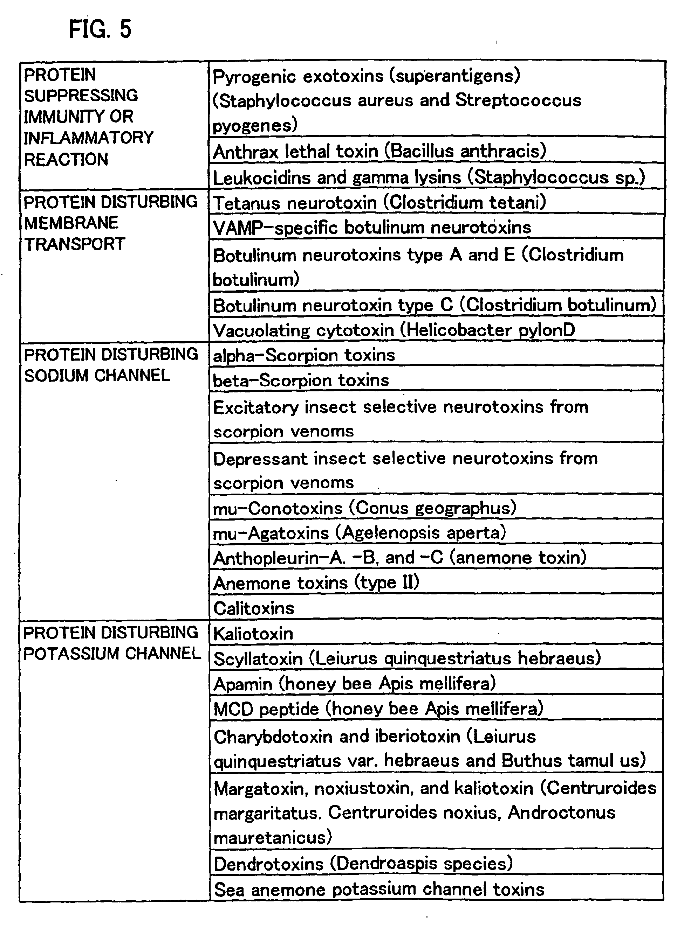

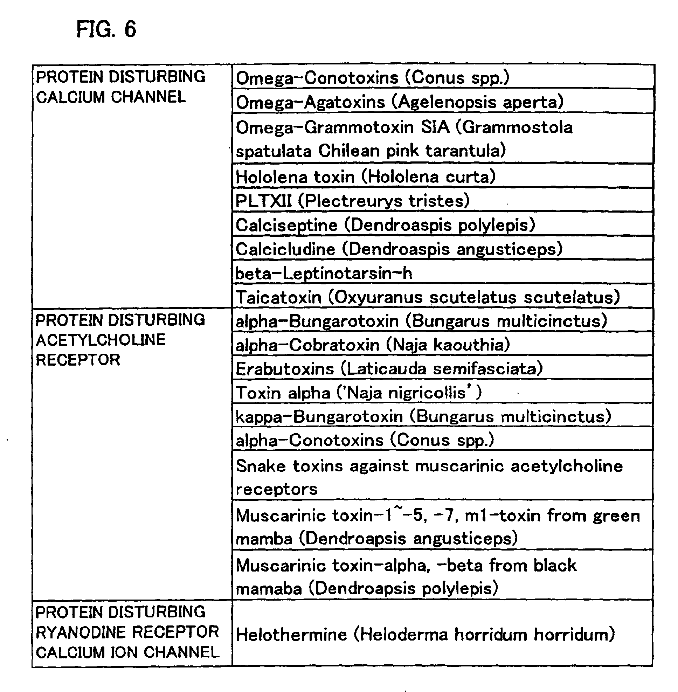

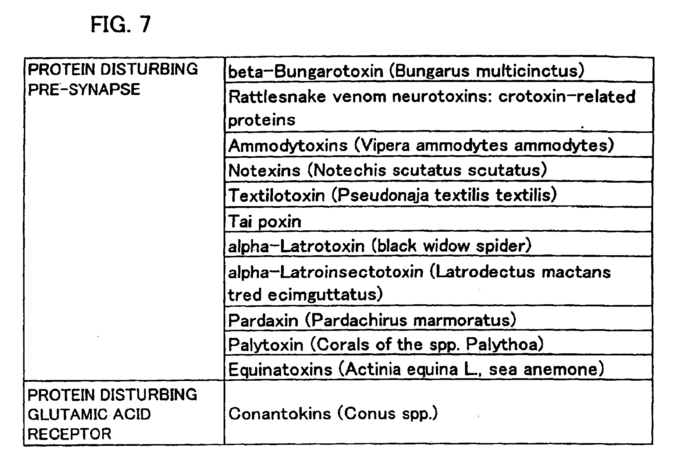

Other examples of the substance to be transferred into a cell include proteins and genes coding the proteins shown in Figure 4 to Figure 7. Further, various cytokines (various interferons, interleukins, and the like) and therapeutic genes, such as tumor suppressor gene (p53, etc.), which are effective to cancer treatment, are applicable.

Further, as a method of transferring such substances to be transferred into a cell to the hollow nanoparticles, various methods generally used in chemical or molecular-biological experimental techniques are applicable. For example, electroporation, sonication, simple diffusion, or use of a charged lipid is preferably exemplified.

Hence, by using such protein hollow nanoparticles or transporters of substances, the specific in vivo or in vitro transfer of substances into cells or tissues are enabled. In addition, as exemplified above for RNase, the use of the protein hollow nanoparticles or the transporters permits the transfer of a substance into particular cells or tissues for treatment of various diseases or as one step of such treatment.

Therapeutic effects of the drug of the present invention were confirmed through animal experiments as will be detailed later in the Examples. In the Examples, the drug of the present invention encapsulating thymidine kinase (HSV1 tk) gene of herpes simplex virus type 1 was administered to nude mice transplanted with cells derived from human squamous cell carcinoma, which was then followed by the administration of ganciclovir (GCV). The sizes of the transplanted cancer tissues were observed to confirm therapeutic effects. The drug was intravenously administered. However, the drug might have been administered by another method including oral, intramuscular, intraperitoneal, and subcutaneous administration.

Hereinafter, embodiments for carrying out the invention are described in further detail through the following Examples and the attached figures. Of course, the invention is not limited in any way by the following examples. It is needless to say that various modifications of the embodiment are allowed.

### EXAMPLES

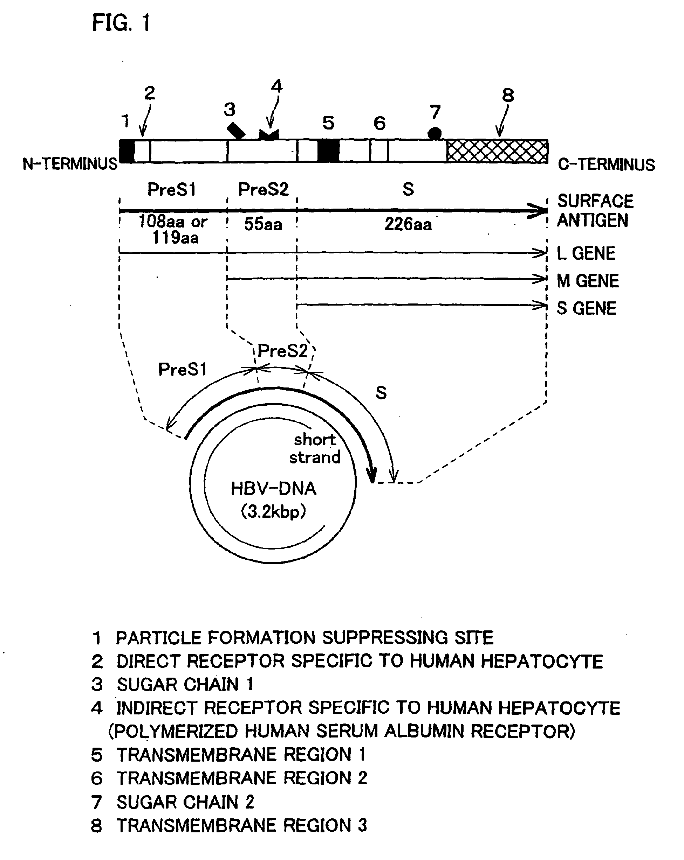

In the following Examples, HBsAg indicates a hepatitis B virus surface antigen. HBsAg, which is a coat protein of HBV, is classified into three types of protein, i.e., S protein, M protein, and L protein, as indicated in the schematic in Figure 1. Among these proteins, the S protein is an important coat protein common to the three proteins. The M protein contains an added 55 amino acids (pre-S2 peptide) at the N-terminus of the S protein. Further, the L protein contains an added 108 or 119 amino acids (pre-S1 peptide) at the N-terminus of the M protein.

The Pre-S regions of the HBsAg L protein (pre-S1, pre-S2) are both known to play an important role in the binding of HBV to the hepatocytes: Pre-S1 has a direct binding site for the hepatocytes, and pre-S2 has a polymerized albumin receptor which binds to the hepatocytes through polymerized albumin in blood.

When HBsAg is expressed in eucaryotic cells, the proteins are expressed and accumulated as membrane proteins on the endoplasmic reticulum membrane. The L protein of HBsAg aggregates between molecules and is released as particles into the lumen side in a budding form, while incorporating the endoplasmic reticulum membrane.

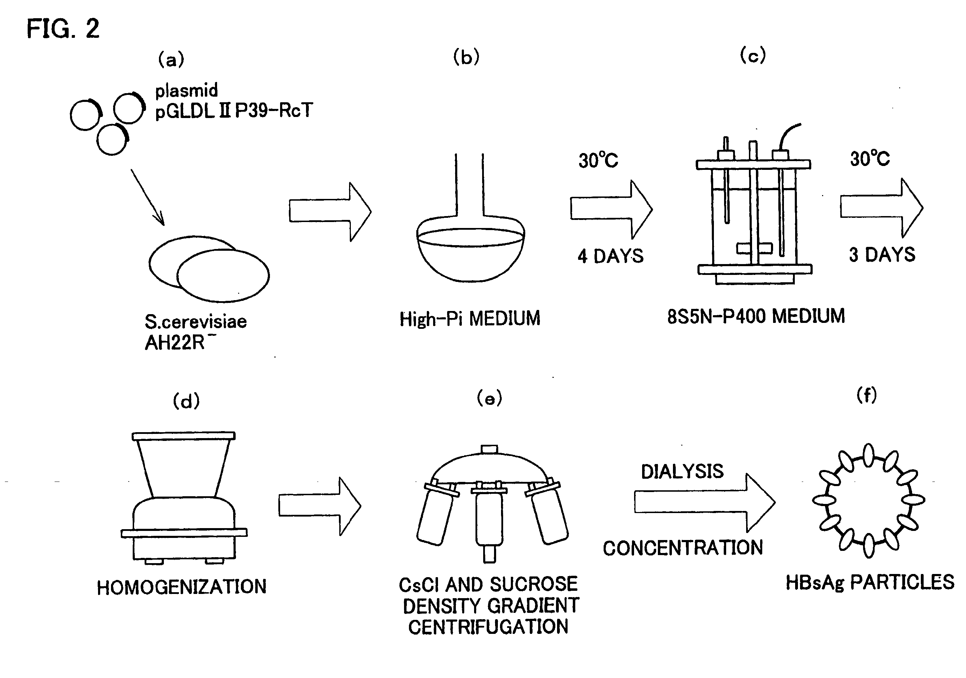

The L protein of HBsAg was used in the following Examples. Figure 2 is a schematic drawing illustrating the expression and purification procedures of HBsAg particles described in the following Examples.

### Example A

### Expression of HBsAg Particles by Recombinant Yeast

The recombinant yeast (Saccharomyces cerevisiae AH22R- strain) carrying pGLDLIIP39-RcT was cultivated in a synthetic medium High-Pi and 8S5N-P400 to express HBsAg L-protein particles, based on the method reported by the present inventors in J. Biol. Chem. Vol. 267, No. 3, 1953-1961 (1992) (Figure 2a to 2c).

From the recombinant yeast in the stationary growth phase (after about 72 hours), whole cell extracts were prepared using Yeast Protein Extraction Reagent (Pierce Chemical Co.), then separated by sodium dodecylsulfate-polyacrylamide gel electrophoresis (SDS-PAGE), and subjected to silver staining to identify HBsAg in the sample.

HBsAg was found to be a protein with a molecular weight of about 52 kDa.

### Example B

### Purification of HBsAg Particles from Recombinant Yeast

The recombinant yeast (wet weight: 26 g) cultivated on the synthetic medium 8S5N-P400 was suspended in 100 ml of Buffer Solution A (7.5 M urea, 0.1 M sodium phosphate, pH 7.2, 15 mM EDTA, 2 mM PMSF, 0.1% Tween 80) and homogenized by a BEAD-BEATER using glass beads. Then, the supernatant was recovered by centrifugation (Figure 2d).

Subsequently, the supernatant was mixed with 0.75-fold volume of 33% (w/w) PEG 6000, and cooled on ice for 30 minutes. Then, the mixture was centrifuged (7,000 rpm, 30 minutes) to recover pellets. The pellets were then re-suspended in Buffer Solution A without Tween 80.

The re-suspended solution was layered over a CsC1 solution of 10-40% gradient and subjected to ultracentrifugation at 28,000 rpm for 16 hours. After centrifugation, the sample was divided into 12 fractions, which were subjected to Western Blotting (primary antibody was anti-HBsAg monoclonal antibody) to identify the fraction containing HBsAg. Further, the fraction containing HBsAg was dialyzed in Buffer Solution A without Tween 80.

(4) The dialysate (12 ml) obtained in (3) was layered over a sucrose of 5-50% gradient and subjected to ultracentrifugation at 28,000 rpm for 16 hours. In the same manner as in (3), the fraction containing HBsAg after centrifugation was identified and dialyzed in Buffer A containing 0.85% NaCl instead of urea and Tween 80. ((2) to (4): Figure 2e)

(5) The procedure of (4) was repeated and the sample after dialysis was concentrated using Ultra Filter Q2000 (Advantech Co.) and refrigerated at 4 degrees Celsius until use. (Figure. 2f)

From the result of Western Blotting (3) after CsC1 density equilibrium centrifugation, HBsAg was found to be a protein with a molecular weight of 52 kDa and an S-antigenicity. A total of about 24 mg of purified HBsAg particles were obtained from 26 g (wet weight) of the fungus body derived from 2.5 L of culture medium.

The fractions obtained in the course of purification were analyzed by silver staining SDS-PAGE. Further, to confirm that protease derived from yeast was removed by purification, the HBsAg particles obtained in (5) was incubated at 37 degrees Celsius for 12 hours, then subjected to SDS-PAGE, and identified by silver staining.

As a result, it was confirmed that protease derived from yeast was completely removed by the overall purification process.

### Example C

### Preparation of a General-purpose Type of HBsAg Particles (HBsAg-Null Particles) for Displaying a Biorecognition Molecule

HBsAg particles are able to specifically infect human hepatocytes, and the hepatocyte-recognizing site, which exhibits high infectivity, displayed on the surface of the particles is reported to be found in the 3rd to 77th amino acid residues of the pre-S1 region (Le Seyec J., Chouteau P., Cannie I., Guguen-Guillouzo C., Gripon P.., J. Virol. March 1999: 73(3), 2052-7).

Here, a method for preparing modified HBsAg particles (hereinafter referred to as HBsAg-Null particles), which lack their high infectivity to hepatocyte and can display any given biorecognition molecule on their surface while maintaining the capability of forming particles will be described.

In the plasmid pGLDLIIP39-RcT described in Example A, in order to eliminate the genetic region coding the human hepatocyte-recognizing site and to introduce the restriction enzyme NotI site (gcggccgc) at the same time, PCR was carried out with QuickChange (TM) Site-Directed Mutagenesis Kit (Stratagene Co.) for the plasmid pGLDLIIP39-RcT, using the oligonucleotide of SEQ ID NO:1 and the oligonucleotide of SEQ ID NO:2 as PCR primers.

Specifically, using Pfu DNA polymerase (Stratagene Co.) as thermostable DNA polymerase, PCR was carried out under the schedule of: denaturation at 95 degrees Celsius for 30 seconds; annealing at 55 degrees Celsius for 1 minute; and polymerization reaction at 68 degrees Celsius for 30 minutes, 30 cycles. Then, the PCR product was treated with restriction enzyme Dpnl, and transformed into Escherichia coli DH5α, after which the vector DNA was extracted from the resulting colony, and the mutated pGLDLIIP39-RcT plasmid (hereinafter, referred to as pGLDLIIP39-RcT (null)) was screened based on its base sequence.

According to the method described in Example A, the plasmid pGLDLIIP39-RcT (null) was transformed and cultivated in synthetic medium High-Pi and in 8S5N-P400, to express HBsAg-Null particles.

From recombinant yeast in the stationary growth phase (72 hours after starting the culture), crude fungus body extracts were prepared using Yeast Protein Extraction Reagent (Pierce Chemical Co.) and then separated by SDS-PAGE, after which HBsAg-Null was identified by silver staining and a Western technique using an anti-S monoclonal antibody (Funakoshi).

Thus, HBsAg-Null was found to be a protein with a molecular weight of about 42 kDa.

In addition, according to the method described in Example B, about 3 mg of purified HBsAg-Null particles was obtained from the fungus body (about 26 g) derived from 2.5 L of the culture medium. Measurement of the S-antigenicity (the degree of particle-formation of HBsAg) with respect to the HBsAg particles and the HBsAg-Null particles using the Auszyme II EIA kit (Dinabot Co., Ltd.) capable of detecting only the HBsAg particle structure gave about equal values for both proteins.

### Example D

### Preparation of HBsAg Particles Displaying an Epidermal Growth Factor (EGF) (HBsAg-EGF Particles)

The EGF receptor is known to be expressed on the surface of various cells, and to be particularly associated with the aggravation of certain cancers (esophageal cancer, colon cancer, etc.). Hence, HBsAg particles targeting the EGF receptor may provide an effective means for the treatment of cancer tissues that express the EGF receptor.

Herein, a method for preparing HBsAg particles of EGF-displaying type (HBsAg-EGF particles) based on the HBsAg-Null particles obtained by the method of Example C is described.

Using a cDNA fragment of EGF precursor of human origin (Bell G I, Fong N M, Stempien M M, Wormsted M A, Caput D, Ku L L, Urdea M S, Rall L B, Sanchez-Pescador R Nucleic Acids Res. Nov. 11, 1986: 14 (21), 8427-46) as a template, a gene fragment coding a mature human EGF region (53 amino acid residue) was amplified by PCR according to conventional methods.

The two PCR primers used were the oligonucleotide of SEQ ID NO:3 for sense and the oligonucleotide of SEQ ID NO:4 for antisense, which were both design to contain the restriction enzyme NotI site (gcggccgc) at the 5'-terminus.

After separation of the PCR product by agarose electrophoresis, the band containing the intended cDNA (about 170 bp) was recovered, and sub-cloned to pCR2.1-TOPO vector (Invitrogen Co.) using TOPO TA Cloning kit (Invitrogen Co.). After confirming the base sequence, this was fragmented with the restriction enzyme NotI to recover the intended DNA fragment of about 170 bp, and using a TaKaRa Ligation kit ver. 2 (TaKaRa Co.), cyclized together with pGLDLIIP39-RcT (null), which was first linearized with the restriction enzyme NotI, after which Escherichia coli DH5α was transformed with the circulated plasmid.

After screening by base sequence analysis, the fused plasmid in which the reading frame of the inserted EGF gene matched that of the HBsAg gene was selected and designated as pGLDLIIP39-RcT-EGF.

According to the method of Example A, the plasmid pGLDLIIP39-RcT-EGF was transformed and cultivated in the synthetic medium High-Pi and 8S5N-P400 to express HBsAg-EGF particles.

From recombinant yeast in the stationary growth phase (72 hours after starting the culture), crude fungus body extracts were prepared using Yeast Protein Extraction Reagent (Pierce Chemical Co.) and then separated by SDS-PAGE, after which HBsAg-EGF was identified by silver staining and a Western technique using an anti-human EGF polyclonal antibody (Santa Cruz Co.).

Thus, HBsAg-EGF was found to be a protein with a molecular weight of about 50 kDa.

According to the method described in Example B, about 3 mg of purified HBsAg-EGF particles was obtained from the fungus body (about 26g) derived from 2.5 L of the culture medium. Measurement of the S-antigenicity HBsAg of particles and HBsAg-EGF particles (the degree of particle-formation of HBsAg) using the Auszyme II EIA kit (Dinabot Co., Ltd.) capable of detecting only the HBsAg particle structure gave about equal values for both proteins.

Thus, it was demonstrated that the HBsAg-EGF particles were obtained, as were the HBsAg particles.

### Example E

### Incorporation of HSV1 tk gene into HBsAg-EGF particles (preparation of HBsAg-EGF particles encapsulating HSV1 tk genes).

Next, kHBsAg-EGF particles encapsulating a HSV1 t gene as the drug of the present invention were prepared by incorporating thymidine kinase (HSV1 tk) gene of herpes simplex virus type 1 as a cancer-treating gene into the HBsAg-EGF particles prepared by the above-mentioned method.

Cancer cells to which the HSV1 tk gene was transferred become sensitive to ganciclovir (GCV), because they express the HSV1 tk gene. Administration of ganciclovir induces fierce collateral effects, killing cancer cells. In this manner, the HSV1 tk gene is one of genes widely used in gene therapy for cancer.

In the present Example, vector pGT 65-hIFN-α (Invivogen Co.), which expresses the HSV1 tk gene, was used to incorporate the HSV1 tk gene into HBsAg-EGF particles. By transferring the expression vector into HBsAg particles by electroporation, particles encapsulating the HSV1 tk gene in HBsAg-EGF particles were prepared. Specifically, 10 µg of the expression vector was introduced to 50 pg of L-protein particles among the HBsAg-EGF particles. A PBS buffer was used in the procedure. Electroporation was conducted at a condition of 220V and 950 µF, using a 4mm cuvette.

### Example F

### Therapeutic effects of HBsAg-EGF particles encapsulating HSV1 tk gene on cancer of nude rat transplanted with human squamous cell carcinoma

Next, therapeutic effects of the HBsAg-EGF particles encapsulating the HSV1 tk gene prepared by the above-mentioned method on human squamous cell carcinoma were confirmed with experimental animals.

In the present Example, as experimental animals, nude rats (strain: F344/NJc1-rnu/rnu, female) purchased from Clea Japan, Inc. were used to prepare tumor bearing rats confirm the therapeutic effects. The tumor bearing rats were prepared by the following method. First, human tumor cell lines (human hepatoma-derived cells HuH-7 (JCRB0403) and human colon carcinoma-derived cells WiDr (ATCC CCL-218) as negative controls) were cultivated, after which 1×10⁶ cells were collected by a conventional method, suspended in HBBS (Hanks BBS; serum-free), and stored in ice. The resulting suspension was mixed with the same amount of Matrigel (40234C from Beckton, Dickinson and Company) as the cells. The mixture was used as instructed in the manual so that the nude rats develops a tumor. The rats were grown for about 3 weeks until the tumor developed into solid cancer of about 2 to 3 cm in diameter, to prepare the tumor bearing rats.

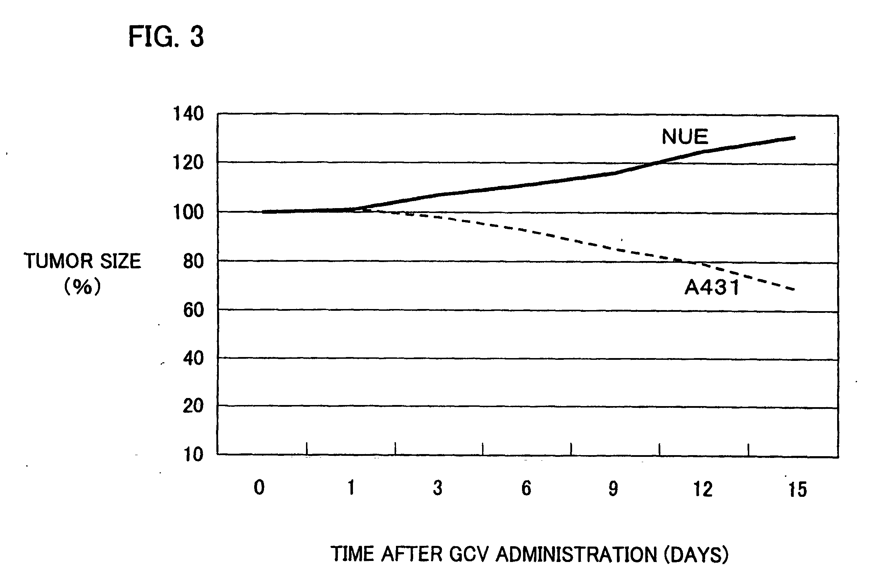

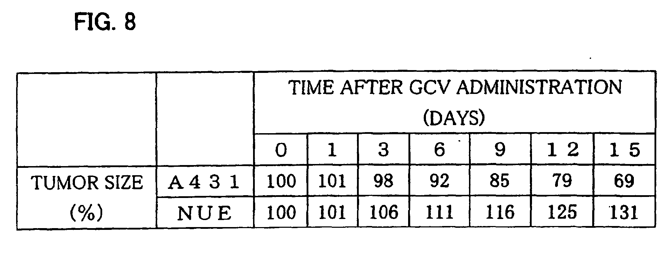

Thereafter, 10 µg of the HBsAg-EGF particles encapsulating the HSV1 tk gene was administered through tail veins of the tumor bearing rats (intravenous injection). The amount of the HBsAg-EGF particles indicates the amount of protein in the HBsAg-EGF particles. The particles are made up of 80% proteins, 10% sugar chains, and 10% phosphor lipid. Five days after the intravenous injection, administration of ganciclovir (GCV) to the tumor bearing rats was started at a rate of 50 mg/kg/day using a osmotic pump (alzet osmotic pump, Cat No. 2ML2). The osmotic pump, together with a drug solution containing GCV, was transplanted subcutaneously to the bilateral dorsal cutis of the tumor bearing rats. The administration of ganciclovir lasted for a maximum of 14 days. After the ganciclovir administration, the condition (size) of the tumor tissues of the tumor bearing rats was observed over time. Specifically, the diameter and length of the tumors were measured with calipers. The measurements were used to evaluate a tumor capacity approximate formula (length x diameter x diameter / 2). Measurement was made on all three rats. Results are shown in Figure 3 and Figure 8.

As shown in Figure 3 and Figure 8, no regression, hence no therapeutic effects, was observed over time in tumor tissues derived from human heptoma (NUE). Meanwhile, in tumor tissues derived from human squamous cell carinoma (A431), regression was observed over time, confirming therapeutic effects of HBsAg-EGF particles encapsulating the HSV1 tk gene which are specific to human squamous cell carcinoma.

In addition, as comparative experiments, the above-mentioned HBsAg-Null particles, which did not display EGF or other growth factors, (encapsulating the HSV1 tk gene) were administered to the same nude rats. Genes were transferred at random, and no specific therapeutic effects, neither to the squamous cell carcinoma or liver cancer, were confirmed.

In this manner, the HBsAg-EGF particles encapsulating the HSV1 tk gene as the drug of the present invention was confirmed to be capable of efficient gene transfer with considerably high specificity to human squamous cells and to have real therapeutic effects on squamous cell carcinoma. Further, through these experiments on experimental animals, a protocol was established to treat squamous cell carcinoma with the drug of the present invention.

The present disclosure includes that contained in the appended claims, as well as that of the foregoing description. Although this invention has been described in its preferred form with a certain degree of particularity, it is understood that the present disclosure of the preferred form has been made only by way of example and that numerous changes in the details of construction and the combination and arrangement of parts may be resorted to without departing from the spirit and the scope of the invention as hereinafter claimed.

### INDUSTRIAL APPLICABILITY

As in the foregoing, the drugs of the present invention is able to specifically and effectively treat diseases of particular cells or tissues through a simple method of intravenous injection. The drugs vastly differs from conventional gene therapy; they do not require surgery. They have extremely low chances of side effects and are straightly applicable in clinical practice.

## Claims

1. A drug, comprising a substance to be transferred into a cell for treatment of a disease encapsulated in a hollow nanoparticle containing a particle-forming protein, the nanoparticle displaying a molecule, such as a growth factor, which binds with a particular molecule on a cell surface.

2. The drug as set forth in claim 1, wherein the protein is a modified hepatitis B virus surface antigen protein.

3. The drug as set forth in either one of claims 1 and 2, wherein the substance to be transferred into a cell is a gene.

4. The drug as set forth in claim 3, wherein the gene is a cancer-treating gene.

5. The drug as set forth in claim 4, wherein the gene is thymidine kinase (HSV1 tk) gene of herpes simplex virus type 1.

6. The drug as set forth in any one of claims 1 through 5, wherein said drug is administered to a human body through intravenous injection.

7. A method of treating a disease through administration of the drug as set forth in any one of claims 1 through 6.


SEQUENCE LISTING

<110> Japan Science and Technology Corporation

<120> Growth factors (or the like) presented protein hollow na
no-particles for therapeutic drug

<130> P023P02

<150> JP 2002-97395
<151> 2002-3-29

<160> 4

<170> PatentIn Ver. 2.1

<210> 1
<211> 39
<212> DNA
<213> Artificial Sequence
<220> Synthesized Oligonucleotide
<400> 1
cgacaaggca tgggaggcgg ccgcagccct caggctcag 39

<210> 2
<211> 39
<212> DNA
<213> Artificial Sequence

<220> Synthesized Oligonucleotide

<400> 2

ctgagcctga gggctgcggc cgcctcccat gccttgtcg 39

<210> 3

<211> 27

<212> DNA

<213> Artificial Sequence

<220> Synthesized Oligonucleotide

<400> 3

ggggcggccg catgaactct gattccg 27

<210> 4

<211> 30

<212> DNA

<213> Artificial Sequence

<220> Synthesized Oligonucleotide

<400> 4

gggcggccgc cacgcagttc ccaccatttc 30